# FASCICULE DE BREVET EUROPEEN

(11) **EP 1 550 435 B1**
(45) Date de publication et mention de la délivrance du brevet: **22.10.2008**
(21) Numéro de dépôt: 04292740.0
(22) Date de dépôt: 19.11.2004
(51) Int. Cl.: A61Q 15/00, A61K 8/26, A61K 8/34, A61K 8/60, A61K 8/90

(54) **Composition déodorante du type émulsion huile-dans-eau contenant un mélange d'alkylpolyglycoside et d'alcool gras et un polyéther polyuréthane non-ionique associatif**
Desodorierende Oel-in-Wasser-Emulsion enthaltend ein Gemisch von Alkylpolyglycosid und Fettalkohol und ausserdem ein nicht ionisches assoziatives Polyether Polyurethan
Deodorizing oil-in-water emulsion comprising a mixture of alkylpolyglycoside and fatty alcohol and a non ionic associative polyether polyurethane

(30) Priorité: 16.12.2003 FR 0351081
(43) Date de publication de la demande: 06.07.2005
(73) Titulaire: L'OREAL S.A., 75008 Paris Cedex (FR)
(72) Inventeur: Aubert, Lionnel, 95270 Asnieres Sur Oise (FR); Douin, Véronique, 78860 Saint-Nom la Bretèche (FR)
(74) Mandataire: Miszputen, Laurent

(56) Documents cités:
- EP-A- 0 606 749
- WO-A-02/44236
- DE-A- 10 208 678
- DE-A- 19 832 425
- US-A- 3 903 232
- US-A- 5 980 874

## Description

L'invention concerne une composition cosmétique comprenant dans un support du type émulsion huile dans eau :
(A) au moins un actif déodorant ;
(B) à titre d'émulsionnant, au moins un mélange comprenant au moins un alkylpolyglycoside dont la chaîne alkyle est linéaire ou ramifiée et comprend de 12 à 22 atomes de carbone et au moins un alcool gras, linéaire ou ramifié, ayant de 12 à 22 atomes de carbone ;
(C) au moins un polyéther polyuréthane non-ionique associatif.

Dans le domaine cosmétique, il est bien connu d'utiliser en application topique, des produits déodorants contenant des substances actives de type anti-transpirant de type bactéricide ou du type absorbeur d'odeurs pour diminuer, voire supprimer, les odeurs axillaires généralement désagréables.

Bien des types différents de compositions déodorantes ont été décrits dans la littérature et sont apparus sur le marché sous des formes telles que des gels, des sticks, des crèmes, des roll-ons ou des aérosols.

Parmi les crèmes déodorantes, il existe trois grands types de formulations : les formulations anhydres ou soft solid, les émulsions eau-dans-huile et les émulsions huile-dans-eau .

Les formulations anhydres soft solid et les émulsions eau-dans-huile qui sont des supports a phase huileuse continue présentent l'inconvénient de produire un toucher gras à l'application et un manque de sensation de fraîcheur voire une efficacité déodorante insuffisante.

Les émulsions huile-dans-eau déodorantes à phase aqueuse continue sont particulièrement recherchées pour leurs qualités cosmétiques originales au niveau de la sensation de fraîcheur sur la peau après application, leur temps de séchage rapide et leur bonne efficacité. Elles contiennent en général des tensioactifs émulsionnants non-ioniques alcool gras ou ester d'acide gras oxyéthyléné et contiennent généralement en plus des agents de consistance pour les épaissir.

On connaît dans les demandes WO92/06778, WO95/13863 et WO98/4761 des systèmes émulsionnants constitués de mélanges d'alkylpolyglucosides et d'alcools gras permettant d'obtenir des émulsions huile-dans-eau pouvant contenir un très large éventail d'actifs et notamment des actifs déodorants. Ces émulsionnants glucolipidiques ont l'avantage d'être compatibles dans tous les types de phases grasses ainsi que dans les milieux aqueux fortement acides notamment comprenant des sels anti-transpirants. Ils permettent d'obtenir des émulsions dans une large gamme de texture, de la richesse à l'évanescence avec des consistances variables, fluides ou crémeuses. Cependant les émulsions huile-dans-eau déodorantes contenant ce type d'émulsionnant présentent des problèmes de stabilité au stockage dans les conditions normales de conservation.

L'ajout d'épaississant classique comme les gommes de guar ne permet pas de résoudre ces problèmes de stabilité au stockage.

Ainsi, on recherche des émulsions huile-dans-eau déodorantes contenant ce type d'émulsionnant glucolipidique qui soient stables pendant le stockage, pouvant être fabriquées dans une large gamme de viscosité et de consistance et présentant une bonne efficacité déodorante. '

La demanderesse a découvert de manière inattendue que cet objectif pouvait être atteint en ajoutant un polyéther polyuréthane non-ionique associatif.

Cette découverte est à la base de la présente invention.

La présente invention a ainsi pour premier objet une composition cosmétique du type émulsion huile dans eau, caractérisée par le fait qu'elle contient :
(A) au moins un actif déodorant ;
(B) à titre d'émulsionnant, au moins un mélange comprenant de 5 à 60 % en poids d'au moins un alkylpolyglycoside dont la chaîne alkyle est linéaire ou ramifiée et comprend de 12 à 22 atomes de carbone de structure telle que définie ci-après et de 95 à 40 % en poids d'au moins un alcool gras, linéaire ou ramifié, ayant de 12 à 22 atomes de carbone par rapport au poids total dudit mélange émulsionnant ;
(C) au moins un polyéther polyuréthane non-ionique associatif.

La présente invention a pour second objet l'utilisation cosmétique d'une composition telle que définie ci-dessus, pour réduire le flux sudoral et/ou masquer, améliorer ou réduire l'odeur désagréable résultant de la décomposition de la sueur humaine par des bactéries.

La présente invention a pour objet également un procédé pour traiter les odeurs axillaires humaines, consistant à appliquer sur la surface axillaire une quantité efficace d'une composition telle que définie précédemment.

Au sens de la présente invention, on entend par "actif déodorant" toute substance capable de réduire le flux sudoral et/ou masquer, absorber, améliorer ou réduire l'odeur désagréable résultant de la décomposition de la sueur humaine par des bactéries.

Au sens de la présente invention, on entend par "polymères associatifs" des polymères hydrophiles capables dans un milieu aqueux, de s'associer réversiblement entre eux ou avec d'autres molécules: Leur structure chimique comprend plus particulièrement au moins une zone hydrophile et au moins une zone hydrophobe.
Par "groupement hydrophobe", on entend un radical ou polymère à chaîne hydrocarbonée, saturée ou non, linéaire ou ramifiée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique ou bien d'un alcool gras polyalkyléné comme le stéareth-100. Il peut également désigner un polymère hydrocarboné tel que par exemple le polybutadiène.

Les compositions conformes à l'invention comportent à titre d'agent émulsionnant au moins un mélange de :
a) au moins un alkylpolyglycoside dont la chaîne alkyle est linéaire ou ramifiée et comprend de 12 à 22 atomes de carbone et
b) au moins un alcool gras, linéaire ou ramifié, ayant de 12 à 22 atomes de carbone.

Au sens de la présente invention, on entend par "alkylpolyglycoside", on entend par alkylmonooside (degré de polymérisation 1) ou alkylpolyoside (degré de polymérisation supérieur à 1).

Le mélange émulsionnant alcool gras/alkylpolyglycoside contient :
(a) de 5 à 60 % en poids d'alkylpolyglycoside(s)
(b) de 95 à 40 % en poids d'alcool(s) gras par rapport au poids total dudit mélange émulsionnant.

Les alkylpolyglycosides peuvent être utilisés seuls ou sous forme de mélanges de plusieurs alkylpolyglycosides. Ils répondent en général à la structure suivante :

**R(O)(G)_{X}**

dans laquelle le radical R est un radical alkyle linéaire ou ramifié en C₁₂-C₂₂ , G est un reste de saccharide et x varie de 1 à 5 , de préférence de 1,05 à 2,5 et plus préférentiellement de 1,1 à 2.

Le reste saccharide peut être choisi parmi glucose, dextrose, saccharose, fructose, gaiactose, maltose, maltotriose, lactose, cellobiose, mannose, ribose, dextrane, talose, allose, xylose, levoglucane, cellulose ou amidon. Plus préférentiellement, le reste saccharide désigne glucose.

Il est en outre à noter que chaque unité de la partie polyoside de l'alkylpolyglycoside peut être sous forme isomérique α ou β, sous forme L ou D et la configuration du reste saccharide peut être de type furanoside ou pyranoside.

Il est bien entendu possible d'utiliser des mélanges d'alkylpolyosides, susceptibles de différer les uns des autres par la nature du motif alkyle porté et/ou la nature de la chaîne polyoside porteuse.

Concernant les alcools gras devant être utilisés, seuls ou en mélanges, en association avec les alkylpolyosides dans les mélanges émulsionnants conformes à l'invention, il peut s'agir d'alcools gras linéaires ou ramifiés, d'origine synthétique, ou encore naturelle comme par exemple les alcools provenant de matières végétales (coprah, palmiste, palme...) ou animales (suif...). Bien entendu, d'autres alcools à longue chaine peuvent également être utilisés, comme par exemple les étheralcools ou bien encore les alcools dits de Guerbet. Enfin, on peut également utiliser certaines coupes plus ou moins longues d'alcools d'origine naturelle, comme par exemple coco (C₁₂ à C₁₆) ou suif (C₁₆ à C₁₈) ou des composés type diols ou cholesterol.

Selon un mode préféré de réalisation de la présente invention, le ou les alcools gras utilisés sont choisis au sein de ceux présentant de 12 à 22 atomes de carbone, et encore plus préférentiellement de 12 à 18 atomes de carbone.

A titre d'exemples particuliers d'alcools gras utilisables dans le cadre de la présente invention, on peut notamment citer l'alcool laurique, cétylique, myristique, stéarylique, isostéarylique, palmitique, oléique, béhénylique, arachidylique qui peuvent donc être pris seuls ou en mélanges

En outre, il est particulièrement avantageux, selon la présente invention, de mettre conjointement en oeuvre un alcool gras et un alkylpolyoside dont la partie alkyle est identique à celle de l'alcool gras retenu.

Les mélanges émulsionnants alcool gras/alkylpolyglycoside tels que définis ci-dessus sont connus en tant que tels. Ils sont décrits dans les demandes WO92/06778, WO95/13863 et WO98/4761 et préparés selon les procédés de préparation indiqués dans ces documents.

Parmi les mélanges alcool gras/alkylpolyglycoside particulièrement préférés, on peut citer les produits vendus par la société SEPPIC sous les appellation MONTANOV ® tels que les mélanges suivants :
Alcool cétylstéarylique/Cocoglucoside- MONTANOV 82®
Alcool arachidylique et alcool béhénylique/arachidylglucoside- MONTANOV 802®
Alcool myristylique/Myristylglucoside - MONTANOV 14 ®
Alcool cétylstéarylique/Cétylstéarylglucoside - MONTANOV 68 ®
Alcool en C₁₄-C₂₂/C₁₂-C₂₀ alkylglucoside - MONTANOV L ®
Cocoalcool /Coco-glucoside - MONTANOV S ®
Alcool isostéarylique/Isostéarylglucoside - MONTANOV WO 18®.

Le mélange alcool gras/alkylpolyglycoside est de préférence présent dans les émulsions conformes à l'invention dans des concentrations allant de 0,5 à 15% en poids et plus préférentiellement de 1 à 10% en poids par rapport au poids total de l'émulsion.

Les polyéthers polyuréthanes non ioniques selon l'invention comportent en général dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.

Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C20 et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention peuvent aussi être choisis parmi ceux décrits dans l'article de G. Fonnum, J. Bakke et Fk. Hansen - Colloid Polym. Sci 271, 380.389 (1993).

Selon une forme particulière de l'invention, on utilisera un polyéther polyuréthane susceptible d'être obtenu par polycondensation d'au moins trois composés comprenant (i) au moins un polyéthylèneglycol comprenant de 150 à 180 moles d'oxyde d'éthylène, (ii) un alcool stéarylique polyoxyéthyléné comprenant 100 moles d'oxyde d'éthylène et (iii) un diisocyanate.

De tels polyéther polyuréthanes sont vendus notamment par la société SASOL SERVO BV sous l'appellation SER-AD FX 1100 ® qui est un polycondensat de polyéthylèneglycol à 136 moles d'oxyde d'éthylène, d'alcool stéarylique polyoxyéthyléné à 100 moles d'oxyde d'éthylène et de hexaméthylène diisocyanate (HDI) ayant poids moléculaire moyen en poids de 30000 (nom INCl : PEG-136/Steareth-1001/SMDI Copolymer).

Selon une autre forme particulière de l'invention on utilisera un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® .

L'ACULYN 46® de nom INCl: PEG-150/Stearyl Alcohol/SMDI Copolymer est un polycondensat de polyéthylèneglycol à 150 ou 180 moles d'oxyde d'éthylène, d'alcool stéarylique et de méthylène bis(4-cyclohexyl-isocyanate) (SMDI) à 15% en poids dans une matrice de maltodextrine (4%) et d'eau (81%)

L'ACULYN 44® PEG-150/Decyl Alcohol/SMDI Copolymer est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%).

La quantité de polyéther(s) polyuréthane(s) associatif(s) en matière active peut aller par exemple de 0,1 à 10 % en poids, de préférence de 0,25 à 8 % en poids et mieux de 1,5 à 5 % en poids par rapport au poids total de la composition.

Parmi les actifs déodorants utilsables selon l'invention, on peut citer les actifs anti-transpirants ou astringents. Ils sont choisis de préférence parmi les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes". De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'aluminium chlorhydrate commercialisé par la société REHEIS sous la dénomination MICRODRY ALUMINUM CHLOROHYDRATE ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF , des sel d'aluminium « activés » par exemple celui commercialisé par la société REHEIS sous la dénomination REACH 103 ou par la société WESTWOOD sous la dénomination WESTCHLOR 200.

Les actifs déodorants peuvent être également des agents bactériostatiques ou des agents bactéricides le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,6,10-triénol (Farnesol) ; les sels d'ammonium quaternaires comme les sels de cetyltrimethylammonium, les sels de cétylpyridinium

Parmi les autres actifs déodorants, on peut citer également les sels de zinc comme le salicylate de zinc, le sulfate de zinc, le chlorure de zinc, le lactate de zinc, le phénolsulfonate de zinc ; la chlorhexidine et les sels; le monocaprate de diglycérol, le monolaurate de diglycérol , monolaurate de glycérol ; les sels de polyhexaméthylène biguanide.

Les actifs déodorants peuvent être présents dans la composition selon l'invention à raison d'environ 0,001 à 40% en poids par rapport à la composition totale, et de préférence à raison d'environ 0,1 à 25% en poids.

La composition cosmétique antitranspirante selon l'invention peut se présenter sous forme de crème plus ou moins épaissie ou "gel solide" distribuée en tube ou en grille ; sous forme de roll-on conditionnée sous forme de bille et contenir à cet égard les ingrédients généralement utilisés dans ce type de produits et bien connus de l'homme de l'art.

La phase huileuse conforme aux émulsions directes selon l'invention comprend un ou plusieurs composés hydrophobes qui rendent ladite phase non-miscible dans l'eau. Ladite phase est liquide à température ambiante (20-25°C). De manière préférentielle, elle est généralement constituée d'une huile ou de mélange d'huiles et comprend au moins 80% de composés ayant une vapeur de pression ne dépassant pas la valeur 4kPa (30 mm Hg) à 25°C.

La phase huileuse contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les huiles émollientes selon l'invention peuvent être choisies parmi des produits du type silicone volatile, des silicones non-volatiles et d'autres émollients non-volatils.

Les silicones volatiles sont définies de façon connue comme des composés volatils à température ambiante. (20-25°C). Elles présentent en général une vapeur de pression à 25°C allant de 1 ou 10 Pa à 2kPa.

On peut citer parmi ces composés les silicones volatiles cycliques et linéaires du type diméthylsiloxane dont les chaines comprennent de 3 à 9 résidus siliconés.

De préférence on choisit les cyclométhicones tétramère (D4), pentamère (D5) ou hexamère (D6).

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid» ; les polyalkylarylsiloxanes comme par exemple les polyméthylphénylsiloxanes, commercialisés par la société Dow Corning sous le nom de « Dow Corning 556 Fluid» ; les copolymères polyéther et siloxane, comme par exemple les diméthicones copolyols.

Parmi les huiles émollientes hydrocarbonées non-volatiles utilisables dans la présente invention, on peut citer par exemple : les dérivés hydrocarbonés liquides comme les huiles minérales et les polyisobutène hydrogénés et plus particulièrement les polydécenes, les paraffines et isoparaffines ayant au moins 10 atomes de carbone , les alcools gras liquides à température ambiante tels que l'alcool isostéarique ou l'octyl dodécanol ; les esters aliphatiques d'alcools en C₃-C₁₈ avec des acides en C₃-C₁₈ comme isopropyl myristate, lauryl myristate, isopropyl palmitate , diisopropyl sebacate, diisopropyl adipate ; les esters aromatiques de l'acide benzoïque avec des alcools en C₁₂-C₁₈ et leurs mélanges comme les alkylbenzoates en C₈-C₁₈ ; les éthers d'alcools gras aliphatiques tels que les éthers d'alcool myristique comme le PPG-3 myristyl éther et les alkyl(C₁-C₄)éthers de polyglycols comme le PPG-4 butyl éther.

Afin d'améliorer l'homogénéité du produit, on peut utiliser des agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité

La phase aqueuse desdites émulsions contient de l'eau et en général d'autres solvants solubles ou miscibles dans l'eau. Les solvants solubles ou miscibles dans l'eau comprennent les mono alcools à chaîne courte par exemple en C₁-C₄ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.
La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans les crèmes, sticks-grilles et roll-ons anti-transpirants.

Les compositions selon l'invention peuvent également contenir également un ou plusieurs autres agents structurants ou gélifiants de la phase huileuse tels que ceux évoqués précédemment dans la description tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010

La composition selon l'invention peut encore être pressurisée et être conditionnée dans un aérosol; elle contient alors les propulseurs généralement utilisés dans ce type de produits et bien connus de l'homme de l'art, comme par exemple le diméthyléther ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon@ et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT.

On peut également utiliser en tant qu'agent propulseur le gaz carbonique, le protoxyde d'azote, l'azote ou l'air comprimé.

L'agent propulseur représente alors de préférence de 20 à 85% en poids du poids La présente invention a également pour objet un procédé cosmétique pour traiter les odeurs axillaires humaines, consistant à appliquer sur la surface axillaire une quantité efficace d'une composition telle que définie ci-dessus.

La présente invention a également pour objet l'utilisation d'au moins un polyéther polyuréthane non-ionique associatif tel que défini ci-dessus dans une émulsion huile-dans-eau comprenant au moins un actif déodorant et au moins un mélange alcool gras/alkylpolyglucoside, dans le but de stabiliser ladite émulsion.

La présente invention a également pour objet un procédé de stabilisation d'une émulsion huile-dans-eau comprenant au moins un actif déodorant et au moins un mélange alcool gras/alkylpolyglucoside , caractérisé par le fait qu'on incorpore dans ladite émulsion au moins un polyéther polyuréthane non-ionique associatif.

Des exemples concrets, mais nullement limitatif, illustrant l'invention vont maintenant être donné.

| **Ingrédients** | **(*)Ex 1** | **(*)Ex** 2 | **(*)Ex3** | **Ex4** | **Ex5** | **Ex6** |
|---|---|---|---|---|---|---|
| Polyéther polyuréthane PEG-136/Steareth-100/HDI (SER-AD- FX1100- SASOL SERVO BV) | - | - | - | 2,00g | - | - |
| Polyéther polyuréthane PEG-150/Stearyl Alcohol/SMDI (ACULYN 46- RHOM & HAAS) | - | | | | 2,00g | 5,00g |
| Hydroxypropyl guar (JAGUAR HP 105- RHODIA) | - | 0,50g | 1,00g | - | - | - |
| Mélange alcool en C₁₄-C₂₂/C₁₂-C₂₀alkylpolyglucoside (MONTANOV L- SEPPIC) | 5,00g | 5,00g | 5,00g | 5,00g | 5,00g | 5,00g |
| Hydroxychlorure d'aluminium en dispersion aqueuse à 50% (CHORHYDROL 50% USP) | 30,0g | 30,0g | 30,0g | 30,0g | 30,0g | 30,0g |
| Diméthicone 200cst (DOW CORNING 200 FLUID) | 7,00g | 7,00g | 7,00g | 7,00g | 7,00g | 7,00g |
| Conservateur | 0,15g | 0,15g | 0,15g | 0,15g | 0,15g | 0,15g |
| Eau qsp | 100 g | 100g | 100g | 100g | 100g | 100g |

| | | | | | | |
|---|---|---|---|---|---|---|
| (*) : hors invention | | | | | | |

Les compositions des exemples 1 à 6 sont préparées selon le mode opératoire suivant :

On introduit dans la cuve de fabrication l'eau et le gélifiant puis on chauffe à 75°C. On réalise l'émulsion en introduisant le mélange MONTANOV L et DC 200 préalablement chauffé à 75°C. On laisse l'agitation turbine et pales pendant 20 mn. On refroidit sous pales uniquement et à 30°C introduire le CHLORHYDROL et le conservateur. On poursuit le refroidissement jusqu'à 25°C.

On observe la stabilité de chacune des formulations 1 à 6
- à 37°C au bout de 2 mois de conservation ;
- à 45°C dans une étuve du type MEMMERT au bout de 2 mois de conservation.

Les résultats de stabilité sont résumés dans le tableau suivant :

| **Stabilité** | **(*)Ex1** | **(*)Ex2** | **(*)Ex3** | **Ex4** | **Ex5** | **Ex6** |
|---|---|---|---|---|---|---|
| Observations après 24 heures à température ambiante | Déphasé | Déphasé | Déphasé | Stable | Stable | Stable |
| Observations après 2 mois à 37°C | Déphasé | Déphasé | Déphasé | Stable | Stable | Stable |
| Observations après 2 mois à 45°C | Déphasé | Déphasé | Déphasé | Stable | Stable | Stable |

Les compositions 4, 5 et 6 selon l'invention comprenant le mélange alcool gras/alkylpolyglucoside et le polyéther polyuréthane restent stables même après 2 mois de conservation à 37 °C et à 45°C contrairement aux émulsions 1, 2 et 3 selon l'art antérieur.

## Revendications

1. Composition cosmétique du type émulsion huile-dans-eau, **caractérisée par le fait qu'**elle contient :
(A) au moins un actif déodorant ;
(B) à titre d'émulsionnant, au moins un mélange comprenant
(i) de 5 à 60 % en poids d'au moins un alkylpolyglycoside répondant à la structure suivante :
R(O)(G)ₓ
dans laquelle le radical R est un radical alkyle linéaire ou ramifié en C₁₂-C₂₂, G est un reste de saccharide et x varie de 1 à 5 , de préférence de 1,05 à 2,5 et plus préférentiellement de 1,1 à 2 ;
(ii) et de 95 à 40 % en poids d'au moins un alcool gras, linéaire ou ramifié, ayant de 12 à 22 atomes de carbone par rapport au poids total dudit mélange émulsionnant ;
(C) au moins un polyéther polyuréthane non-ionique associatif.

2. Composition selon la revendication 1, dans laquelle le reste G saccharide est choisi parmi glucose, dextrose, saccharose, fructose, galactose, maltose, maltotriose, lactose, cellobiose, mannose, ribose, dextrane, talose, allose, xylose, levoglucane, cellulose ou amidon.

3. Composition selon la revendication 2, dans laquelle le reste G saccharide désigne glucose.

4. Composition selon l'une quelconque des revendications 1 à 3, où le ou les alcools gras présentent de 12 à 18 atomes de carbone.

5. Composition selon la revendication 4, dans laquelle le ou les alcools gras sont choisis seuls ou en mélanges, parmi l'alcool laurique, cétylique, myristique, stéarylique, isostéarylique, palmitique, oléique, béhénylique, arachidylique.

6. Composition selon l'une quelconque des revendications 1 à 5, où l'alkylpolyglucoside présente une partie alkyle identique à celle de l'alcool gras.

7. Composition selon l'une quelconque des revendications 1 à 6, où le mélange alcool gras/alkylpolyglycoside est choisi parmi les mélanges suivants :
Alcool cétylstéarylique/Cocoglucoside ;
Alcool arachidylique et alcool béhénylique/Arachidylglucoside ;
Alcool myristylique/Myristylglucoside ;
Alcool cétylstéarylique/Cétylstéarylglucoside ;
Alcool en C₁₄-C₂₂/C₁₂-C₂₀ alkylglucoside ;
Cocoalcool /Coco-glucoside ;
Alcool isostéarylique/Isostéarylglucoside.

8. Composition selon l'une quelconque des revendications 1 à 7, où le mélange alcool gras/alkylpolyglycoside est choisi parmi
Alcool cétylstéarylique/Cétylstéarylglucoside ;
Alcool en C₁₄-C₂₂/C₁₂-C₂₀ alkylglucoside.

9. Composition selon l'une quelconque des revendications 1 à 8, où le mélange alcool gras/alkylpolyglycoside est présent dans des concentrations allant de 0,5 à 15% en poids et de préférence de 1 à 10% en poids par rapport au poids total de la composition.

10. Composition selon l'une quelconque des revendications 1 à 9, où le polyéther polyuréthane non ionique associatif comporte dans sa chaîne, à la fois des séquences hydrophiles et des séquences hydrophobes qui peuvent être des enchaînements aliphatiques seuls et/ou des enchaînements cycloaliphatiques et/ou aromatiques.

11. Composition selon la revendication 10, où le polyéther polyuréthane non ionique associatif comporte au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile.

12. Composition selon la revendication 11, où le polyéther polyuréthane non ionique associatif comporte une chaîne hydrocarbonée à un bout ou aux deux bouts d'une séquence hydrophile.

13. Composition selon l'une quelconque des revendications 10 à 12, où le polyéther polyuréthane non ionique associatif est multiséquencé, en particulier sous forme de tribloc.

14. Composition selon la revendication 13, où le polyéther polyuréthane non ionique associatif est sous forme de tribloc.

15. Composition selon la revendication 14, où le polyéther polyuréthane non ionique associatif est sous forme de tribloc dont la séquence hydrophile est une chaîne polyoxyéthyténée comportant de 50 à 1000 groupements oxyéthylénés.

16. Composition selon l'une quelconque des revendications 10 à 15, où le polyéther polyuréthane non ionique associatif est susceptible d'être obtenu par polycondensation d'au moins trois composés comprenant (i) au moins un polyéthylèneglycol comprenant de 150 à 180 moles d'oxyde d'éthylène, (ii) un alcool stéarylique polyoxyéthyléné comprenant 100 moles d'oxyde d'éthylène et (iii) un diisocyanate.

17. Composition selon la revendication 16, où le polyéther polyuréthane non ionique associatif est le copolymère PEG-136/Steareth-100/SMDI.

18. Composition selon l'une quelconque des revendications 10 à 17, où le polyéther polyuréthane non ionique associatif est susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

19. Composition selon la revendication 18, où le polyéther polyuréthane non ionique associatif est le copolymère PEG-150/Stearyl Alcohol/SMDI Copolymer.

20. Composition selon la revendication 18, où le polyéther polyuréthane non ionique associatif est le copolymère PEG-150/Decyl Alcohol/SMDI.

21. Composition selon l'une quelconque des revendications 1 à 20, où le ou les polyéther polyuréthane non ionique associatif sont présents dans une quantité allant de 0,1 à 10 % en poids, de préférence de 0,25 à 8 % en poids et mieux de 1,5 à 5 % en poids par rapport au poids total de la composition.

22. Composition selon l'une quelconque des revendications 1 à 21, où l'actif déodorant est choisi parmi les actifs anti-transpirants.

23. Composition selon la revendication 22, où l'actif anti-transpirant est choisi parmi les sels d'aluminium et/ou de zirconium ; le bicarbonate de sodium ; les complexes d'hydroxychlorure de zirconium et d'hydroxychlorure d'aluminium avec un acide aminé

24. Composition selon la revendication 23, où l'actif anti-transpirant est choisi parmi le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate.

25. Composition selon la revendication 24, où l'actif anti-transpirant est le chlorhydrate d'aluminium.

26. Composition selon la revendication 23, où l'actif anti-transpirant est choisi parmi les complexes d'hydroxychlorure de zirconium et d'hydroxychlorure d'aluminium avec la glycine (ZAG).

27. Composition selon la revendication 26, où l'actif anti-transpirant est choisi parmi l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

28. Composition selon l'une quelconque des revendications 1 à 21, où l'actif déodorant est choisi parmi les agents bactériostatiques ou des agents bactéricides.

29. Composition selon la revendication 28, où l'actif déodorant est choisi parmi le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,6,10-triénol (Farnesol) ; les sels d'ammonium quaternaires.

30. Composition selon l'une quelconque des revendications 1 à 21, où l'actif déodorant est choisi parmi les sels de zinc ; la chlorhexidine et les sels; le monocaprate de diglycérol, le monolaurate de diglycérol, monolaurate de glycérol les sels de polyhexaméthylène biguanide.

31. Composition selon l'une quelconque des revendications 1 à 30, où l'actif déodorant est présent à raison d'environ 0,001 à 40% en poids par rapport à la composition totale, et de préférence à raison d'environ 0,1 à 25% en poids.

32. Composition selon l'une quelconque des revendications 1 à 31, **caractérisée par le fait qu'**elle se présente sous forme de crème plus ou moins épaissie distribuée en tube ou en grille ; sous forme de roll-on.

33. Composition selon l'une quelconque des revendications 1 à 32, **caractérisée par le fait que** la phase huileuse contient une ou plusieurs huiles émollientes siliconée ou hydrocarbonée, volatiles ou non-volatiles.

34. Composition selon l'une quelconque des revendications 1 à 33, **caractérisée par le fait qu'**elle contient en plus au moins un agent de suspension.

35. Composition selon l'une quelconque des revendications 1 à 34, **caractérisée par le fait qu'**elle contient en plus au moins une poudre organique.

36. Composition selon l'une quelconque des revendications 1 à 35, **caractérisée par le fait qu'**elle contient en plus des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants.

37. Composition selon l'une quelconque des revendications 1 à 36, **caractérisée par le fait qu'**elle contient en plus un ou plusieurs agents structurants ou gélifiants de la phase huileuse

38. Composition selon l'une quelconque des revendications 1 à 37, **caractérisée par le fait qu'**elle est pressurisée et conditionnée dans un aérosol et qu'elle contient un ou plusieurs agents propulseurs.

39. Utilisation non-thérapeutique cosmétique d'une composition selon l'une quelconque des revendications 1 à 38, pour réduire le flux sudoral et/ou masquer, améliorer ou réduire l'odeur désagréable résultant de la décomposition de la sueur humaine par des bactéries.

40. Procédé non-thérapeutique pour traiter les odeurs axillaires humaines, consistant à appliquer sur la surface axillaire une quantité efficace d'une composition selon l'une quelconque des revendications 1 à 38.

41. Utilisation d'au moins un polyéther polyuréthane non-ionique associatif tel que défini dans l'une quelconque des revendications précédentes dans une émulsion huile-dans-eau comprenant au moins un actif déodorant et au moins un mélange alcool gras/alkylpolyglucoside tel que défini dans l'une quelconque des revendications précédentes, dans le but de stabiliser ladite émulsion.

42. Procédé de stabilisation d'une émulsion huile-dans-eau comprenant au moins un actif déodorant et au moins un mélange alcool gras/alkylpolyglucoside tel que défini dans l'une quelconque des revendications précédentes, **caractérisé par le fait qu'**on incorpore dans ladite émulsion au moins un polyéther polyuréthane non-ionique associatif tel que défini dans l'une quelconque des revendications précédentes.

## Claims

1. Cosmetic composition of the oil-in-water emulsion type, **characterized in that** it comprises:
(A) at least one deodorant active principle;
(B) as emulsifier, at least one mixture comprising:
(i) from 5 to 60% by weight of at least one alkyl polyglycoside corresponding to the following structure:
R(O)Gₓ
in which the R radical is a linear or branched C₁₂-C₂₂ alkyl radical, G is a saccharide residue and x varies from 1 to 5, preferably from 1.05 to 2.5 and more preferably from 1.1 to 2;
(ii) and from 95 to 40% by weight of at least one linear or branched fatty alcohol having from 12 to 22 carbon atoms, with respect to the total weight of the said emulsifying mixture;
(C) at least one nonionic associative polyether polyurethane.

2. Composition according to Claim 1, in which the saccharide residue G is chosen from glucose, dextrose, sucrose, fructose, galactose, maltose, maltotriose, lactose, cellobiose, mannose, ribose, dextran, talose, allose, xylose, levoglucan, cellulose or starch.

3. Composition according to Claim 2, in which the saccharide residue G denotes glucose.

4. Composition according to any one of Claims 1 to 3, where the fatty alcohol or alcohols exhibit from 12 to 18 carbon atoms.

5. Composition according to Claim 4, in which the fatty alcohol or alcohols are chosen, alone or as mixtures, from lauryl, cetyl, myristyl, stearyl, isostearyl, palmityl, oleyl, behenyl or arachidyl alcohol.

6. Composition according to any one of Claims 1 to 5, where the alkyl polyglucoside exhibits an alkyl part identical to that of the fatty alcohol.

7. Composition according to any one of Claims 1 to 6, where the fatty alcohol/alkyl polyglycoside mixture is chosen from the following mixtures:
Cetearyl alcohol/Coco glucoside;
Arachidyl alcohol and behenyl alcohol/Arachidyl glucoside;
Myristyl alcohol/Myristyl glucoside;
Cetearyl alcohol/Cetearyl glucoside;
C₁₄-C₂₂ Alcohol/C₁₂-C₂₀ Alkyl glucoside;
Coconut alcohol/Coco glucoside;
Isostearyl alcohol/Isostearyl glucoside.

8. Composition according to any one of Claims 1 to 7, where the fatty alcohol/alkyl polyglycoside mixture is chosen from:
Cetearyl alcohol/Cetearyl glucoside;
C₁₄-C₂₂ Alcohol/C₁₂-C₂₀ Alkyl glucoside.

9. Composition according to any one of Claims 1 to 8, where the fatty alcohol/alkyl polyglycoside mixture is present in concentrations ranging from 0.5 to 15% by weight and preferably from 1 to 10% by weight, with respect to the total weight of the composition.

10. Composition according to any one of Claims 1 to 9, where the nonionic associative polyether polyurethane comprises, in its chain, both hydrophilic blocks and hydrophobic blocks which can be aliphatic sequences alone and/or cycloaliphatic and/or aromatic sequences.

11. Composition according to Claim 10, where the nonionic associative polyether polyurethane comprises at least two lipophilic hydrocarbon chains, having from 6 to 30 carbon atoms, separated by a hydrophilic block, it being possible for the hydrocarbon chains to be pendent chains or chains at the ends of the hydrophilic block.

12. Composition according to Claim 11, where the nonionic associative polyether polyurethane comprises a hydrocarbon chain at one end or at both ends of a hydrophilic sequence.

13. Composition according to any one of Claims 10 to 12, where the nonionic associative polyether polyurethane is multiblock, in particular in the triblock form.

14. Composition according to Claim 13, where the nonionic associative polyether polyurethane is in the triblock form.

15. Composition according to Claim 14, where the nonionic associative polyether polyurethane is in the triblock form, the hydrophilic block of which is a polyoxyethylene chain comprising from 50 to 1000 oxyethylene groups.

16. Composition according to any one of Claims 10 to 15, where the nonionic associative polyether polyurethane is capable of being obtained by polycondensation of at least three compounds comprising
(i) at least one polyethylene glycol comprising from 150 to 180 mol of ethylene oxide, (ii) a polyoxyethylenated stearyl alcohol comprising 100 mol of ethylene oxide and (iii) a diisocyanate.

17. Composition according to Claim 16, where the nonionic associative polyether polyurethane is the copolymer PEG-136/Steareth-100/SMDI.

18. Composition according to any one of Claims 10 to 17, where the nonionic associative polyether polyurethane is capable of being obtained by polycondensation of at least three compounds comprising
(i) at least one polyethylene glycol comprising from 150 to 180 mol of ethylene oxide, (ii) stearyl alcohol or decyl alcohol and (iii) at least one diisocyanate.

19. Composition according to Claim 18, where the nonionic associative polyether polyurethane is the copolymer PEG-150/Stearyl Alcohol/SMDI Copolymer.

20. Composition according to Claim 18, where the nonionic associative polyether polyurethane is the copolymer PEG-150/Decyl Alcohol/SMDI.

21. Composition according to any one of Claims 1 to 20, where the nonionic associative polyether polyurethane or polyurethanes are present in an amount ranging from 0.1 to 10% by weight, preferably from 0.25 to 8% by weight and better still from 1.5 to 5% by weight, with respect to the total weight of the composition.

22. Composition according to any one of Claims 1 to 21, where the deodorant active principle is chosen from antiperspirant active principles.

23. Composition according to Claim 22, where the antiperspirant active principle is chosen from aluminium and/or zirconium salts; sodium bicarbonate; complexes of zirconium hydroxychloride and of aluminium hydroxychloride with an amino acid.

24. Composition according to Claim 23, where the antiperspirant active principle is chosen from aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulphate, aluminium zirconium octachlorohydrate, aluminium zirconium pentachlorohydrate, aluminium zirconium tetrachlorohydrate or aluminium zirconium trichlorohydrate.

25. Composition according to Claim 24, where the antiperspirant active principle is aluminium chlorohydrate.

26. Composition according to Claim 23, where the antiperspirant active principle is chosen from complexes of zirconium hydroxychloride and of aluminium hydroxychloride with glycine (ZAG).

27. Composition according to Claim 26, where the antiperspirant active principle is chosen from aluminium zirconium octachlorohydrex GLY, aluminium zirconium pentachlorohydrex GLY, aluminium zirconium tetrachlorohydrate GLY and aluminium zirconium trichlorohydrate GLY.

28. Composition according to any one of Claims 1 to 21, where the deodorant active principle is chosen from bacteriostatic agents or bactericidal agents.

29. Composition according to Claim 28, where the deodorant active principle is chosen from 2,4,4'-trichloro-2'-hydroxydiphenyl ether (triclosan), 2,4-dichloro-2'-hydroxydiphenyl ether, 3',4',5'-trichlorosalicylanilide, 1-(3',4'-dichlorophenyl)-3-(4'chlorophenyl)urea (triclocarban) or 3,7,11-trimethyldodeca-2,6,10-trienol (farnesol); or quaternary ammonium salts.

30. Composition according to any one of Claims 1 to 21, where the deodorant active principle is chosen from zinc salts; chlorhexidine and its salts; diglycerol monocaprate, diglycerol monolaurate or glycerol monolaurate; or polyhexamethylene biguanide salts.

31. Composition according to any one of Claims 1 to 30, where the deodorant active principle is present in a proportion of approximately 0.001 to 40% by weight, with respect to the total composition, and preferably in a proportion of approximately 0.1 to 25% by weight.

32. Composition according to any one of Claims 1 to 31, **characterized in that** it is provided in the form of a more or less thickened cream dispensed in a tube or in a twist stick; in the form of a roll on.

33. Composition according to any one of Claims 1 to 32, **characterized in that** the oily phase comprises one or more volatile or nonvolatile and emollient silicone or hydrocarbon oils.

34. Composition according to any one of Claims 1 to 33, **characterized in that** it additionally comprises at least one suspending agent.

35. Composition according to any one of Claims 1 to 34, **characterized in that** it additionally comprises at least one organic powder.

36. Composition according to any one of Claims 1 to 35, **characterized in that** it additionally comprises cosmetic adjuvants chosen from waxes, softeners, antioxidants, opacifiers, stabilizers, moisturizing agents, vitamins, fragrances, bactericides, preservatives, polymers or thickening agents.

37. Composition according to any one of Claims 1 to 36, **characterized in that** it additionally comprises one or more structuring or gelling agents for the oily phase.

38. Composition according to any one of Claims 1 to 37, **characterized in that** it is pressurized and packaged in an aerosol and **in that** it comprises one or more propellants.

39. Nontherapeutic cosmetic use of a composition according to any one of Claims 1 to 38 for reducing the flow of sweat and/or masking, improving or reducing the unpleasant odour resulting from the decomposition of human sweat by bacteria.

40. Nontherapeutic method for treating human axillary odours, consisting in applying, to the axillary surface, an effective amount of a composition according to any one of Claims 1 to 38.

41. Use of at least one nonionic associative polyether polyurethane as defined in any one of the preceding claims in an oil-in-water emulsion comprising at least one deodorant active principle and at least one fatty alcohol/alkyl polyglucoside mixture as defined in any one of the preceding claims with the aim of stabilizing the said emulsion.

42. Method for the stabilization of an oil-in-water emulsion comprising at least one deodorant active principle and at least one fatty alcohol/alkyl polyglucoside mixture as defined in any one of the preceding claims, **characterized in that** at least one nonionic associative polyether polyurethane as defined in any one of the preceding claims is incorporated in the said emulsion.

## Patentansprüche

1. Kosmetische Zusammensetzung vom Öl-in-Wasser-Typ, **dadurch gekennzeichnet, dass** sie enthält:
(A) mindestens einen desodorierenden Wirkstoff;
(B) als Emulgator zumindest ein Gemisch enthaltend
(i) 5 bis 60 Gew.-% mindestens eines Alkylpolyglycosids der folgenden Struktur:
R(O)(G)ₓ
worin die Gruppe R eine lineare oder verzweigte Alkylgruppe mit 12 bis 22 Kohlenstoffatomen bedeutet, G ein Saccharidrest ist und x im Bereich von 1 bis 5, vorzugsweise 1,05 bis 2,5 und noch bevorzugter 1,1 bis 2 liegt; und
(ii) 95 bis 40 Gew.-% mindestens eines linearen oder verzweigten Fettalkohols mit 12 bis 22 Kohlenstoffatomen, bezogen auf das Gesamtgewicht des Emulgatorgemisches und
(C) mindestens ein assoziatives nichtionisches Polyetherpolyurethan.

2. Zusammensetzung nach Anspruch 1, bei der der Saccharidrest G unter Glucose, Dextrose, Saccharose, Fructose, Galactose, Maltose, Maltotriose, Lactose, Cellobiose, Mannose, Ribose, Dextran, Talose, Allose, Xylose, Levoglucan, Cellulose oder Stärke ausgewählt ist.

3. Zusammensetzung nach Anspruch 2, bei der der Saccharidrest G die Glucose bedeutet.

4. Zusammensetzung nach einem der Ansprüche 1 bis 3, wobei der Fettalkohol oder die Fettalkohole 12 bis 18 Kohlenstoffatome aufweisen.

5. Zusammensetzung nach Anspruch 4, wobei der oder die Fettalkohole einzeln oder in Form von Gemischen unter Laurylalkohol, Cetylalkohol, Myristylalkohol, Stearylalkohol, Isostearylalkohol, Palmitylalkohol, Oleylalkohol, Behenylalkohol und Arachidonalkohol ausgewählt sind.

6. Zusammensetzung nach einem der Ansprüche 1 bis 5, wobei das Alkylpolyglucosid einen Alkylbereich aufweist, der mit dem des Fettalkohols identisch ist.

7. Zusammensetzung nach einem der Ansprüche 1 bis 6, wobei das Gemisch Fettalkohol/Alkylpolyglycosid unter den folgenden Gemischen ausgewählt ist:
Cetylstearylalkohol/Cocoglucosid;
Arachidonalkohol und Behenylalkohol/Arachidylglucosid;
Myristylalkohol/ Myristylglucosid;
Cetylstearylalkohol/ Cetylstearylglucosid;
C₁₄-₂₂-Alkohol/ C₁₂₋₂₀-Alkylglucosid;
Cocosalkohol/ Cocosglucosid;
Isostearylalkohol/Isostearylglucosid.

8. Zusammensetzung nach einem der Ansprüche 1 bis 7, wobei das Gemisch Fettalkohol/Alkylpolyglycosid ausgewählt ist unter:
Cetylstearylalkohol/ Cetylstearylglucosid;
C₁₄₋₂₂-Alkohol/ C₁₂₋₂₀-Alkylglucosid.

9. Zusammensetzung nach einem der Ansprüche 1 bis 8, wobei das Gemisch Fettalkohol/Alkylpolyglycosid in Konzentrationen von 0,5 bis 15 Gew.-% und vorzugsweise 1 bis 10 Gew.-%, bezogen auf das Gesamtgewicht der Zusammensetzung, enthalten ist.

10. Zusammensetzung nach einem der Ansprüche 1 bis 9, wobei das assoziative nichtionische Polyetherpolyurethan in seiner Kette gleichzeitig hydrophile Sequenzen und hydrophobe Sequenzen aufweist, bei denen es sich um aliphatische Verknüpfungen alleine und/ oder cycloaliphatische Verknüpfungen und/ oder aromatische Verknüpfungen handeln kann.

11. Zusammensetzung nach Anspruch 10, wobei das assoziative nichtionische Polyetherpolyurethan mindestens zwei lipophile Kohlenwasserstoffketten mit 6 bis 30 Kohlenstoffatomen aufweist, die über eine hydrophile Sequenz getrennt sind, wobei die Kohlenwasserstoffketten Seitenketten oder Ketten am Ende der hydrophilen Sequenz sein können.

12. Zusammensetzung nach Anspruch 11, wobei das assoziative nichtionische Polyetherpolyurethan eine Kohlenwasserstoffkette an einem Ende oder an beiden Enden einer hydrophilen Sequenz aufweist.

13. Zusammensetzung nach einem der Ansprüche 10 bis 12, wobei das assoziative nichtionische Polyetherpolyurethan multisequentiell vorliegt, insbesondere in Dreiblockform.

14. Zusammensetzung nach Anspruch 13, wobei das assoziative nichtionische Polyetherpolyurethan in Dreiblockform vorliegt.

15. Zusammensetzung nach Anspruch 14, wobei das assoziative nichtionische Polyetherpolyurethan in Dreiblockform vorliegt, wobei die hydrophile Sequenz eine Polyoxyethylenkette ist, die 50 bis 1 000 Oxyethylengruppen aufweist.

16. Zusammensetzung nach einem der Ansprüche 10 bis 15, wobei das assoziative nichtionische Polyetherpolyurethan durch Polykondensation von zumindest drei Verbindungen erhältlich ist, die (i) mindestens ein Polyethylenglycol mit 150 bis 180 mol Ethylenoxid, (ii) einen polyethoxylierten Stearylalkohol mit 100 mol Ethylenoxid und (iii) ein Diisocyanat umfassen.

17. Zusammensetzung nach Anspruch 16, wobei das assoziative nichtionische Polyetherpolyurethan das Copolymer PEG-136/Steareth-100/SMDI ist.

18. Zusammensetzung nach einem der Ansprüche 10 bis 17, wobei das assoziative nichtionische Polyetherpolyurethan durch Polykondensation von zumindest drei Verbindungen erhältlich ist, die (i) zumindest ein Polyethylenglycol mit 150 bis 180 mol Ethylenoxid, (ii) Stearylalkohol oder Decylalkohol und (iii) mindestens ein Diisocyanat umfassen.

19. Zusammensetzung nach Anspruch 18, wobei das assoziative nichtionische Polyetherpolyurethan das Copolymer PEG-150/Stearylalkohol/SMDI ist.

20. Zusammensetzung nach Anspruch 18, wobei das assoziative nichtionische Polyetherpolyurethan das Copolymer PEG-150/Decylakohol/SMDI ist.

21. Zusammensetzung nach einem der Ansprüche 1 bis 20, wobei das oder die assoziativen nichtionischen Polyetherpolyurethane in einer Menge von 0,1 bis 10 Gew.-%, vorzugsweise 0,25 bis 8 Gew.-% und besser 1,5 bis 5 Gew.-%, bezogen auf das Gesamtgewicht der Zusammensetzung, enthalten sind.

22. Zusammensetzung nach einem der Ansprüche 1 bis 21, wobei der desodorierende Wirkstoff unter den Antitranspirantien ausgewählt ist.

23. Zusammensetzung nach Anspruch 22, wobei der schweißverhütende Wirkstoff unter den Salzen von Aluminium und/oder Zirconium; Natriumbicarbonat; und Komplexen von Zirconiumhydroxychlorid und Aluminiumhydroxychlorid und einer Aminosäure ausgewählt ist.

24. Zusammensetzung nach Anspruch 23, wobei der schweißverhütende Wirkstoff ausgewählt ist unter Aluminiumhydroxychlorid, Aluminium Chlorohydrex, Aluminium Chlorohydrex PEG, Aluminium Chlorohydrex PG, Aluminium Dichlorohydrate, Aluminium Dichlorohydrex PEG, Aluminium Dichlorohydrex PG, Aluminium Sesquichlorohydrate, Aluminium Sesquichlorohydrex PEG, Aluminium Sesquichlorohydrex PG, Alaunsalzen, Aluminiumsulfat, Aluminium Zirconium Octachlorohydrate, Aluminium Zirconium Pentachlorohydrate, Aluminium Zirconium Tetrachlorohydrate, Aluminium Zirconium Trichlorhydrate.

25. Zusammensetzung nach Anspruch 24, wobei es sich bei dem schweißverhütenden Wirkstoff um das Aluminiumhydroxychlorid handelt.

26. Zusammensetzung nach Anspruch 23, wobei der schweißverhütende Wirkstoff unter den Komplexen von Zirconiumhydroxychlorid und Aluminiumhydroxychlorid mit Glycin (ZAG) ausgewählt ist.

27. Zusammensetzung nach Anspruch 26, wobei der schweißverhütende Wirkstoff unter Aluminium Zirconium Octachlorohydrex GLY, Aluminium Zirconium Pentachlorohydrex GLY, Aluminium Zirconium Tetrachlorohydrex GLY und Aluminium Zirconium Trichlorohydrate-GLY ausgewählt ist.

28. Zusammensetzung nach einem der Ansprüche 1 bis 21, wobei der desodorierende Wirkstoff unter den bakteriostatischen Wirkstoffen oder den bakteriziden Wirkstoffen ausgewählt ist.

29. Zusammensetzung nach Anspruch 28, wobei der desodorierende Wirkstoff unter 2,4,4'-Trichlor-2'-hydroxydiphenylether (Triclosan), 2,4-Dichlor-2'-hydroxydiphenylether, 3',4',5'-Trichlorsalicylanilid, 1-(3',4'-Dichlorphenyl)-3-(4'-chlorphenyl)-harnstoff (Triclocarban) oder 3,7,11-Trimethyldodeca-2,6,10-trienol (Farnesol); und quartären Ammoniumsalzen ausgewählt ist.

30. Zusammensetzung nach einem der Ansprüche 1 bis 21, wobei der desodorierende Wirkstoff unter den Zinksalzen; Chlorhexidin und seinen Salzen; Diglycerylmonocaprat, Diglycerylmonolaurat, Glycerylmonolaurat; und den Salzen von Polyhexamethylenbiguanid ausgewählt ist.

31. Zusammensetzung nach einem der Ansprüche 1 bis 30, wobei der desodorierende Wirkstoff in einer Menge von etwa 0,001 bis 40 Gew.-%, bezogen auf die gesamte Zusammensetzung, und vorzugsweise in einer Menge von etwa 0,1 bis 25 Gew.-% vorliegt.

32. Zusammensetzung nach einem der Ansprüche 1 bis 31, **dadurch gekennzeichnet, dass** sie in Form einer mehr oder weniger dickflüssigen Creme, die aus einer Tube oder über ein Gitter abgegeben wird; in Form eines Roll-ons vorliegt.

33. Zusammensetzung nach einem der Ansprüche 1 bis 32, **dadurch gekennzeichnet, dass** die Ölphase ein oder mehrere weichmachende Siliconöle oder Kohlenwasserstofföle enthält, die flüchtig oder nichtflüchtig sind.

34. Zusammensetzung nach einem der Ansprüche 1 bis 33, **dadurch gekennzeichnet, dass** sie ferner mindestens ein Suspendiermittel enthält.

35. Zusammensetzung nach einem der Ansprüche 1 bis 34, **dadurch gekennzeichnet, dass** sie ferner mindestens ein organisches Pulver enthält.

36. Zusammensetzung nach einem der Ansprüche 1 bis 35, **dadurch gekennzeichnet, dass** sie ferner kosmetische Adjuvantien enthält, die unter den Wachsen, beruhigenden Stoffen, Antioxidantien, Trübungsmitteln, Stabilisatoren, Hydratisierungsmitteln, Vitaminen, Parfums, Bakteriziden, Konservierungsmitteln, Polymeren, Parfums und Verdickungsmitteln ausgewählt sind.

37. Zusammensetzung nach einem der Ansprüche 1 bis 36, **dadurch gekennzeichnet, dass** sie ferner ein oder mehrere Strukturiermittel oder Gelbildner für die Ölphase enthält.

38. Zusammensetzung nach einem der Ansprüche 1 bis 37, **dadurch gekennzeichnet, dass** sie in einer Aerosolvorrichtung konfektioniert und unter Druck gesetzt ist und ein oder mehrere Treibmittel enthält.

39. Nichttherapeutische kosmetische Verwendung einer Zusammensetzung nach einem der Ansprüche 1 bis 38, um den Schweißfluss zu vermindern und/ oder zu verdecken, den unangenehmen Geruch zu verbessern oder zu vermindern, der durch die Zersetzung des menschlichen Schweißes durch Bakterien entsteht.

40. Nichttherapeutisches Verfahren zur Behandlung von menschlichem Achselgeruch, das darin besteht, auf die Oberfläche der Achseln eine wirksame Menge einer Zusammensetzung nach einem der Ansprüche 1 bis 38 aufzubringen.

41. Verwendung mindestens eines assoziativen nichtionischen Polyetherpolyurethans, wie es in einem der vorhergehenden Ansprüche definiert ist, in einer Öl-in-Wasser-Emulsion, die mindestens einen desodorierenden Wirkstoff und zumindest ein Fettalkohol/Alkylpolyglucosid-Gemisch, wie es in einem der vorhergehenden Ansprüche definiert ist, enthält, um die Emulsion zu stabilisieren.

42. Verfahren zur Stabilisierung einer Öl-in-Wasser-Emulsion, die mindestens einen desodorierenden Wirkstoff und zumindest ein Fettalkohol/Alkylpolyglucosid-Gemisch, wie in einem der vorhergehenden Ansprüche definiert ist, enthält, **dadurch gekennzeichnet, dass** in die Emulsion mindestens ein assoziatives nichtionisches Polyetherpolyurethan eingearbeitet wird, wie es in einem der vorhergehenden Ansprüche definiert ist.
